# EUROPEAN PATENT APPLICATION

(11) **EP 2 283 830 A1**
(43) Date of publication of application: **16.02.2011**
(21) Application number: 09100365.7
(22) Date of filing: 23.07.2009
(51) Int. Cl.: A61K 9/48

(54) **Aqueous enteric capsule coating**

(71) Applicant: Actogenix N.V., 9052 Zwijnaarde (BE)
(72) Inventor: The designation of the inventor has not yet been filed

(57) **Abstract**

The invention relates generally to the field of delayed release dosage forms for the delivery and release of active ingredients into the intestine. Particularly oral dosage forms characterized in being stable at lower pH and in having an accelerated dissolution profile at higher pH.

## Description

### Field of the Invention

The invention relates generally to the field of delayed release dosage forms for the delivery and release of active ingredients into the intestine. Particularly oral dosage forms characterized in being stable at lower pH and in having an accelerated dissolution profile at higher pH.

### Background to the Invention

Oral formulations often comprise drugs or other active ingredients, such as beneficial bacteria, which for optimal results should be delivered in digestive compartments downstream of the stomach. In order to achieve this, delayed release dosage forms were developed which consist of a formulation containing the active ingredient, surrounded by an enteric coating. These coatings are designed such that they are stable at low pH values, such as in the stomach, and only start disintegrating at higher pH values further down the digestive tract.

The best known compounds used for these enteric coatings comprise copolymers such as for example the water-soluble copolymers [methacrylic acid - ethyl acrylate] and [methacrylic acid - methyl acrylate - methyl methacrylate]; and the water-insoluble copolymers [methacrylic acid - methyl methacrylate (50/50)] and [methacrylic acid - methyl methacrylate (30/70)]. These copolymers are resistant to low pH values of the stomach and have different pH solubility (being 5.5, 7.0, 6.0 and 7.0 respectively) in later compartments of the digestive tract.

The objective of the present invention was to provide an enteric capsule coating, that is resistant to low pH values in the stomach and in the upper part of the intestine at pH values of about 5.5 but that will disintegrate rapidly further on in the digestive tract at pH values of about 6.8. As pilot experiments indicated that coatings with water-insoluble polymers were brittle and resulted in high residues of organic solvents such as isopropanol, it was a further objective of the invention to provide an aqueous coating with the desired disintegration profile, but with decreased brittleness.

Aqueous enteric capsule coatings are know from the art. For example, US7094425 provides enteric capsule coatings comprising the water-soluble copolymer [methacrylic acid - ethyl acrylate] or [methacrylic acid - methyl acrylate - methyl methacrylate]. The copolymer [methacrylic acid - ethyl acrylate], however, is designed to only dissolve from pH 7.0, whereas the copolymer [methacrylic acid - methyl acrylate - methyl methacrylate] is designed to dissolve from pH 5.5. Therefore, coating with either one of these copolymers alone, does not result in the desired disintegration profile for the coatings of the present invention (resistance at pH values of about 5.5 and rapid disintegration at pH values of about 6.8).

Furthermore, it is known from the art that mixing of different polymers at different ratios can be used (Assmus et al., US2004/0142035A1) for fine-tuning the resistance of the coating to certain pH values. For example Assmus et al. used varying concentrations of EUDRAGIT® FS30D and EUDRAGIT® L30D-55 for coating granules. A 50:50 ratio of EUDRAGIT® FS30D and EUDRAGIT® L30D-55 resulted in a very fast release (100% within 1 hour) at pH 5.5. Higher ratios of EUDRAGIT® FS30D (80/20 and 85/15), were required to obtain stability at pH values below 6.0 and 6.5 respectively and fast release (more than 50% within 1 hour) at pH 6.5 and 6.8 respectively. Contrary to these results, a 50:50 ratio of EUDRAGIT® FS30D and EUDRAGIT® L30D-55 (US2004/0142035 A1) applied on beads/pellets was protective (less that 55% release) for over 3 hours at pH 6.0 and 6.5.

These seemingly contradicting results indicate that not only the composition of the enteric coating but also the used formulation (e.g. granules versus beads/pellets) is highly relevant for the release profile of the compositions. Indeed, as demonstrated by Gupta et al. (Gupta et al., Biomaterials, 21(2000) 1115-1119), the release rate of diclofenac sodium from beads was found to be slower in comparison to the release from microgranules. These observations indicate that for each formulation the optimal ratio of polymers should be determined with regard to the desired release profile.

So in conclusion, there was a need for an optimized enteric coating for capsules, which has a high mechanical strength and which is resistant to pH values of about and below 5.5 and rapidly disintegrates at pH values of about 6.8.

### Detailed Description of the Invention

In a first embodiment, the present invention provides an enteric capsule coating comprising a mixture at a ratio of between and about 50/50 - 45/55 of a first copolymer consisting of [methacrylic acid - ethyl acrylate]; and second copolymer consisting of [methacrylic acid - methyl acrylate - methyl methacrylate]. These copolymers are commercially available from Evonik Pharma Polymers (former Röhm Pharma) in Germany whereby the copolymer of [methacrylic acid - ethyl acrylate] is commonly known as EUDRAGIT® L30D-55 and hereinafter also referred to as the 'first copolymer'; and the copolymer of [methacrylic acid - methyl acrylate - methyl methacrylate] is commonly known as EUDRAGIT® FS30D, hereinafter also referred to as the 'second copolymer'.

The enteric capsule coating further comprises a plasticizer, and optionally a lubricant, an emulsifier, or both.

The addition of a plasticizer to the formulation is contemplated as being within the scope of the invention. Plasticizers reduce the minimum film-forming temperature as well as the glass transition temperature. Furthermore, the addition of a plasticizer in a polymeric film system increases the flexibility of the film coatings and is therefore necessary for the formation of smooth films that are free of cracks and other defects. Plasticizers function by weakening the intermolecular attractions between the polymer chains. These additives have been shown to influence various polymer properties, including the mechanical, adhesive, and drug-release characteristics.

Plasticizers useful in the invention include, by way of example and without limitation, triethylcitrate, tributyl citrate, acetyltriethylcitrate, acetyltributylcitrate, dibutyl sebacate, glyceryl triacetate, polyethylene glycol, propylene glycol, glycerol and citric acid; in particular triethylcitrate.

The amount of plasticizer used in the formulation will depend upon its composition, physical properties, effect upon the copolymer, interaction with other components of the formulation, ability to solubilize the therapeutic compound or other factors to be considered in the preparation of pharmaceutical formulations. The amount of plasticizer present in the formulation affects its properties. By way of example, when the plasticizer is triethyl citrate, its content will generally not exceed about 20% wt. of the formulation.

When present, the relative amount of plasticizer used may be expressed by the ratio high molecular weight copolymer % wt.: plasticizer % wt., and will generally fall in the range of about 100:0 to about 80:20. The amount of plasticizer will generally not exceed the amount of copolymer.

Lubricants are not required in order to practice the invention. However, as polymer solutions and dispersions go through a tacky phase during drying, lubricants may be added to the coating solution in order to avoid agglomeration and to prevent the film coatings from becoming sticky.

Lubricants useful in the invention include, by way of example and without limitation, talc, magnesium stearate, colloidal silicon dioxide, precipitated silicon dioxide and glycerol monostearate, in particular glycerol monostearate.

The amount of lubricant used in the formulation will depend upon its composition, physical properties, effect upon the copolymer, interaction with other components of the formulation, ability to solubilize the therapeutic compound or other factors to be considered in the preparation of pharmaceutical formulations. The amount of lubricant present in the formulation affects its properties. By way of example, when the lubricant is glycerol monostearate, its content will generally not exceed about 10% wt. of the formulation.

When present, the relative amount of lubricant used may be expressed by the ratio high molecular weight copolymer % wt.: lubricant % wt., and will generally fall in the range of about 100:0 to about 90:10.

Emulsifiers are not required in order to practice the invention. They can, however, be useful in increasing the solubility of the different components of the formulation, as they prevent oily components, such as for example certain lubricants, from separating from the watery environment of the composition.

Emulsifiers useful in the invention include, by way of example and without limitation, sodium laurylsulfate, polysorbates, propylene glycols, fatty acids, oils, fatty alcohols, macrogol cetostearyl ether, in particular polysorbate 80.

The amount of emulsifier used in the formulation will depend upon its composition, physical properties, effect upon the copolymer, interaction with other components of the formulation, ability to solubilize the therapeutic compound or other factors to be considered in the preparation of pharmaceutical formulations. The amount of emulsifier present in the formulation affects its properties. By way of example, when the emulsifier is polysorbate 80, its content will generally not exceed about 5% wt. of the formulation.

When present, the relative amount of emulsifier used may be expressed by the ratio high molecular weight copolymer % wt.: emulsifier % wt., and will generally fall in the range of about 100:0 to about 95:5. The amount of emulsifier will generally not exceed the amount of copolymer.

In a particular embodiment, the present invention provides an enteric capsule coating comprising a mixture at a ratio of between and about 45/55 to 50/50 of a first copolymer [methacrylic acid - ethyl acrylate]; and a second copolymer [methacrylic acid - methyl acrylate - methyl methacrylate]; between and about 2 and 20% wt of trietylcitrate; between and about 1 and 20% wt of glycerol monostearate; and between and about 0,5 and 5% wt of polysorbate 80. All concentrations are based on dry polymer substance.

In a more particular embodiment, the present invention provides an enteric capsule coating comprising a mixture of about 50/50 of a first copolymer [methacrylic acid - ethyl acrylate]; and a second copolymer [methacrylic acid - methyl acrylate - methyl methacrylate]; about 8% wt of trietylcitrate; about 5% wt of glycerol monostearate; and about 2% wt of polysorbate 80. All concentrations are based on dry polymer substance.

The present invention further provides a capsule coated with an enteric capsule coating as defined in the present invention.

For the intestinal delivery of active ingredients, the enteric coated capsules of the present invention should be stable at low pH (up to pH 5.5) and in having an accelerated dissolution profile at higher pH (above pH 5.5). The optimal release is realized when the capsules disintegrate at a pH of about 6.8 within 1 hour. Thus in a further aspect of the present invention the capsules are coated with an enteric capsule coating characterized in being stable at low pH (up to pH 5.5) and in disintegrating at a pH of about 6.8 within 1 hour. In particular said capsules are coated with an enteric capsule coating as defined herein.

The capsule used in the aforementioned formulation is typically selected from the group consisting of a gelatin capsule, a starch capsule, a hydroxypropylmethylcellulose (HPMC) capsule and the like; in particular a HPMC capsule. Compared to gelatin capsules, the latter have better coating properties (the smooth surface of the gelatin capsules can lead to shell embritlement and poor adhesion of the enteric coating), and can be filled using standard and widely available capsule filling machines.

In a further embodiment of the present invention, the capsules are sealed after filling in the overlapping region of the capsule body and cap by commonly known sealing techniques like banding or applying a sealing and/or heat to the gap between capsule body and cap. Preferred is a banding in which a banding solution, which may include a solvent, is applied individually and uniformly to the external edge of the gap of a capsule to be sealed to form a liquid ring around the circumference of the capsule. Removing excess banding solution from the exterior of the capsule and drying the banding before coating will prevent problems e. g. with non-uniformity of the coating at the gap or development of fissures during storage under stressing conditions, which can lead to an unwanted loss of gastro resistant properties. This sealing further increases the capsule's resistance to fluid infiltration and allows coating at a lower density when compared to non-sealed capsules. The latter is beneficial in the optimization of the desired characteristics of the enteric coated capsules of the present invention. The enteric coating is typically applied in the range of 5 - 20 mg/cm2 of capsule surface, in particular from 5-10 mg/ cm², more in particular at 5, 7.5 or 10 mg/ cm².

The enteric-coated capsules of the present invention are particularly useful for the intestinal delivery of viable bacteria, including recombinant bacteria; in particular lactic acid bacterial strains, selected from the group consisting of *Streptococcus, Lactococcus, Lactobacillus* or *Bifidobacterium*; more in particular *lactococcus lactis.*

This invention will be better understood by reference to the Experimental Details that follow, but those skilled in the art will readily appreciate that these are only illustrative of the invention as described more fully in the claims that follow thereafter. Additionally, throughout this application, various publications are cited. The disclosure of these publications is hereby incorporated by reference into this application to describe more fully the state of the art to which this invention pertains.

### EXAMPLE: Optimization of Enteric coating

The following example illustrates the invention. Other embodiments will occur to the person skilled in the art in light of these examples.

The objective of this experiment was to determine the optimal coating composition and density for calciumhydrogenphosphate-filled HPMC (hydroxypropylmethyl cellulose) capsules in order to obtain the following characteristics:
- stable for at least 2 hours in 0.1 N HCl
- stable for at least 1 hour in buffer at pH 5.5
- disintegration within 1 hour in buffer at pH6.8

A combination of the water-soluble polymers EUDRAGIT® FS30D and EUDRAGIT® L30D-55 in different mixing ratios was used. At the same time the impact of the addition of different amounts of plasticizer (TEC = triethylcitrat) based on polymer dry weight (wt/wt) and the addition of glidant (Talc, Glycerolmonostearate (GMS) and Imwitor 900K) was evaluated. For all compositions, Polysorbate 80 (33 % aqueous solution) was added as an emulsifier. Capsules were coated in a Brucks Modell VII coating pan and removed from the pan, at different coating densities ranging from 5 - 20 mg/cm².

The composition and the coating density for each tested batch is given in table 1.

**Table 1. Composition and coating densities for the different batches**

| **Batch** | **FS30D/ L30D-55** | **Talcum** | **GMS** | **TEC (% wt/wt)** | **mg/cm²** |
|---|---|---|---|---|---|
| 692/34 | 85/15 | + | | 5 | 20 |
| 692/35 | 85/15 | + | | 5 | 10 |
| 692/36 | 75/25 | + | | 5 | 5-10 |
| 692/38 | 75/25 | | + | 5 | 5, 6.5, 7.5, 10 |
| 692/39 | 75/25 | | + | 8 | 5, 6.5 |
| 692/40 | 65/35 | | + | 8 | 5, 7.5 |
| 692/41 | 55/45 | | + | 8 | 5, 7.5 |
| 692/43 | 50/50 | | + | 8 | 5, 7.5 |

| | | | | | |
|---|---|---|---|---|---|
| TEC= Trietyl citrate GMS= glycerol monostearate | | | | | |

After coating, capsules were used in a disintegration experiment where they were transferred from one buffer into the other, as indicated below. The disintegration time was recorded as the moment on which the capsule started to lose powder.
- 2 hours in 0.1 N HCl
- 1 hour at pH 5.5 phosphate buffer
- 1 hour at pH 6.8 phosphate buffer

Results for the disintegration test are given in table 2.

**Table 2. Disintegration test results**

| **Batch** | **FS30D/ L30D-55** | **0.1 N HCl** | **pH 5.5** | **pH 6.8** | **mg/cm²** |
|---|---|---|---|---|---|
| 692/34 | 85/15 | 0% | 0% | 0% | 20 |
| 692/35 | 85/15 | 0% | 0% | 0% | 10 |
| 692/36 | 75/25 | 0% | 0% | 16% | 5 |
| 692/36 | 75/25 | 0% | 0% | 33% | 7.5 |
| 692/36 | 75/25 | 0% | 0% | 33% | 10 |
| 692/38 | 75/25 | 0% | 0% | 33% | 5 |
| 692/38 | 75/25 | 0% | 0% | 33% | 6.5 |
| 692/38 | 75/25 | 0% | 0% | 0% | 7.5 |
| 692/39 | 75/25 | 0% | 0% | 0% | 5 |
| 692/39 | 75/25 | 0% | 0% | 0% | 6.5 |
| 692/40 | 65/35 | 0% | 0% | 50% | 5 |
| 692/40 | 65/35 | 0% | 0% | 0% | 7.5 |
| 692/41 | 55/45 | 0% | 0% | 83% | 5 |
| 692/41 | 55/45 | 0% | 0% | 83% | 7.5 |
| 692/43 | 50/50 | 0% | 16% | 100% | 5 |
| 692/43 | 50/50 | 0% | 0% | 100% | 7.5 |

| | | | | | |
|---|---|---|---|---|---|
| % represents % of capsule that lost powder (= complete disintegration) at the indicated pH values | | | | | |

As is evident from table 2, batches 692/41 (55/45 ratio) and 692/43 (50/50) give the best results with regard to the desired disintegration profile. These batches are resistant (less than 20% disintegration) to low pH values and are highly sensitive (more than 80% disintegration) to pH values of 6.8. The other batches are also clearly resistant to lower pH values, however they are also mostly resistant to pH 6.8. From these batches, only batch 692/40 (65/35) also gives a reasonable disintegration at pH 6.8 (50%) when applied at a coating density of 5 mg/cm².

The exact composition used for the enteric coating of the batches 692/41 and 692/43 is given in table 3.

**Table 3. Composition of enteric coatings (% wt/wt)**

| **Batch** | **L30D-55** | **FS30D** | **TEC** | **GMS** | **Polysorbate 80** | **Total** |
|---|---|---|---|---|---|---|
| 692/41 | 9,3 mg 39% | 11,3 mg 47% | 1,85 mg 8% | 1,02 mg 4,3% | 0,41 mg 1,7% | 23,9 mg 100% |
| 692/43 | 15,37 mg 43% | 15,37 mg 43% | 2,76 mg 8% | 1,53 mg 4,3% | 0,62 mg 1,7% | 35,65 mg 100% |

## Claims

1. An enteric capsule coating **characterized in** comprising a mixture at a ratio of between and about 50/50 - 45/55 of a first copolymer [methacrylic acid - ethyl acrylate]; and a second copolymer [methacrylic acid - methyl acrylate - methyl methacrylate].

2. The enteric capsule coating according to claim 1 further containing a plasticizer, a lubricant, an emulsifier, or any combination thereof

3. The enteric capsule coating according to claim 2 wherein, the plasticizer is selected from triethylcitrate, acetyltriethylcitrate, acetyltributyl-citrate, dibutyl sebacate, glyceryl triacetate, tributyl citrate, polyethylene glycol, propylene glycol, glycerol and citric acid; in particular triethylcitrate.

4. The enteric capsule coating according to claim 2, wherein the lubricant is selected from talc, magnesium stearate, colloidal silicon dioxide, precipitated silicon dioxide, and glycerol monostearate, in particular glycerol monostearate.

5. The enteric capsule coating according to claim 2, wherein the emulsifier is selected from sodium laurylsulfate, polysorbates, propylene glycols, fatty acids, oils, fatty alcohols, macrogol cetostearyl ether, in particular polysorbate 80.

6. The enteric capsule coating according to anyone of claims 1 to 5 **characterized in** comprising an equal amount of said first and second copolymer wherein each of said copolymers represents between and about 30 and 50% wt; between and about 5 and 10% wt of a plasticizer, in particular about 8 %wt of trietylcitrate; between and about 2 and 5% wt of a lubricant, in particular about 4% wt of glycerol monostearate; and between and about 0,5 and 2% wt of an emulsifier, in particular about 2% wt of polysorbate 80.

7. A capsule coated with an enteric capsule coating as defined in anyone of claims 1 to 6.

8. The capsule according to claim 7, wherein the capsule is selected from the group consisting of a gelatin capsule, a starch capsule, a hydroxypropylmethylcellulose (HPMC) capsule and the like; in particular a HPMC capsule.

9. The capsule according to any one of claims 7 and 8, further **characterized by** having a sealing at the overlapping region of the capsule's body and cap.

10. The coated capsule according to any one of claims 7 to 9 wherein the enteric coating is applied in the range of 5 - 10 mg/cm² of capsule surface.
